# EUROPEAN PATENT APPLICATION

(11) **EP 2 253 612 A1**
(43) Date of publication of application: **24.11.2010**
(21) Application number: 10161362.8
(22) Date of filing: 12.04.2006
(51) Int. Cl.: C07C 277/08, C07C 279/18, A61K 9/19, A61K 31/24

(54) **Organic compounds**

(30) Priority: 14.04.2005 GB 0507577
(62) Divisional of application: 06742574.4
(71) Applicant: Novartis AG, 4056 Basel (CH); Novartis Pharma GmbH, 1230 Wien (AT); IRM LLC, Hamilton, HM 11 (BM)
(72) Inventor: Danahay, Henry Luke, Horsham, Sussex RH 12 5AB (GB); Legrand, Darren Mark, Horsham, Sussex RH12 5AB (GB); Tully, David C., San Diego, CA 92130 (US); Harris, Jennifer Leslie, San Diego, CA 92129 (US); Heuerding, Silvia, 4051, Basel (CH); Singh, Dilraj, 4058, Basel (CH); Maas, Janet Catherine, Horsham, Sussex RH 12 5AB (GB); Roettele, Juergen, 79112, Freiburg (DE); Reber, Jean-Louis, 68680, Kembs (FR); Monnier, Stéphanie, 68190, Raedersheim (FR)
(74) Representative: von Sprecher, Georg

(57) **Abstract**

The invention describes novel pharmaceutically acceptable salt forms of camostat, processes for lyophilisation, taste-masked formulations, nebulised formulations and the use of each of the fore-going in the treatment of respiratory diseases, particularly cystic fibrosis and chronic obstructive pulmonary disease (COPD).

## Description

This invention relates to organic compounds, salts, formulation and processes and their use as pharmaceuticals.

The invention provides, in one aspect, the use of a serine protease inhibitor for the preparation of a medicament for the treatment of a disease mediated by inhibition of a channel activating protease (CAP). The disease is suitably a respiratory disease, more suitably selected from cystic fibrosis and chronic obstructive pulmonary disease (COPD).

Examples of suitable serine proteases, the inhibitors of which are implicated in the uses of the present invention, include trypsin, matriptase, prostasin (PRSS8), plasmin, tPA, uPA, Xa, IXa, thrombin, tissue factor, compliment factors, tryptase, HNE, kallikrein (plasma and tissue), matriptase and TRMPSS 3 and 4.

Thus, as a first aspect, the invention provides the use of a serine protease inhibitor, e.g. an inhibitor of a serine protease selected from trypsin, matriptase, prostasin (PRSS8), plasmin, tPA, uPA, Xa, IXa, thrombin, tissue factor, compliment factors, tryptase, HNE, kallikrein (plasma and tissue), matriptase and TRMPSS 3 and 4, in the manufacture of medicament for the treatment of a disease mediated by inhibition of a channel activating protease (CAP), e.g. a respiratory disease, most suitably cystic fibrosis or COPD.

As a further aspect of the present invention, the invention provides the use of a Factor Xa inhibitor in the manufacture of a medicament for the treatment of a disease mediated by inhibition of a channel activating protease (CAP), e.g. a respiratory disease, most suitably cystic fibrosis or COPD. Suitable Factor Xa inhibitors for use in the present invention include fondaparin sodium, rivaroxaban, idrapainux sodium, apixaban and otamixaban and those compounds specifically and generally described in US6469036, particularly RWJ-58643 (J&J), US6022861, US6211154, particularly MLN-1021 (Millenium), FR2773804, e.g. SR123781 (Sanofi-Aventis), DE19829964, e.g. tanogitran, US6469026, WO0001704, e.g. BIBR-1109 (Boehringer Ingelheim), DE19829964, e.g. BIBT-0871, BIBT-1011 and BIBT-0932CL (Boehringer Ingelheim) and DE19816983. Other FactorXa inhibitors for use in the present invention include those compounds specifically disclosed in the review document Expert Opin. Ther. Patents (2006) 16(2):119-145, e.g. DX-9065a, DPC-423, Razaxaban, BAY59-7938 and compounds number 5-153.

As a further aspect of the present invention, there is provided the use of a thrombin inhibitor in the manufacture of a medicament for the treatment of a disease mediated by inhibition of a channel activating protease (CAP), e.g. a respiratory disease, most suitably cystic fibrosis or chronic obstructive pulmonary disease (COPD). Suitable thrombin inhibitors for use in the present invention include argatroban, glycyrrhizin (Ligand), odiparcil, corthrombin, those compounds specifically and generally described in US5523308 (J&J), WO9102750, e.g. Hirulog-1 (Biogen), DE19706229, e.g. dabigratan and dabigratan etexilate, AU8551553, e.g. efegatran sulfate hydrate, WO9311152, e.g. inogatran, US2003134801, e.g. LB-30870 (LG Chem), Org42675 (Akzo Nobel), EP559046, e.g. napsagatran, WO0170736, e.g. SSR-182289, EP615978, e.g. S-18326 (Servier), WO9513274, e.g. UK-156406 (Pfizer), EP0918768, e.g. AT-1362 (C&C Research Labs), WO0055156, e.g. AT-1459 (C&C Research Labs), JP1999502203, e.g. BCH-2763 (Nat Res Council of Canada), EP623596, e.g. BMS-189090 (BMS), CA2151412, e.g. BMS-191032 (BMS), US5037819, e.g. BMY-43392-1 (BMS), GB2312674, e.g. CGH-1484A (Novartis), EP739886, e.g. CI-1028, LB-30057 and PD-172524 (LG Chem), DE4115468, e.g. CRC-220 (Dade Behring Marburg), AU8817332, e.g. DuP-714 (BMS), JP96333287, e.g. F-1070 (Fuji Yakuhin), WO9701338, e.g. L-373890, L-374087 and L-375052 (Merck), WO9740024, e.g. L-375378 (Merck), WO9842342, e.g. L-376062 (Merck), WO0251824, e.g. LK-658 and LK-732 (Lek), WO9705160, e.g. LR-D/009 (Guidotti), EP479489, e.g. LY-293435 (Lilly), AU8945880, e.g. MDL-28050 (Sanofi Avenits), EP195212, e.g. MDL-73756 (Sanofi Avenits), AU9059742, e.g. MDL-74063 (Sanofi Avenits), JP90289598, e.g. Cyclotheonamide A, WO9965934, e.g. NAPAP-PS (Organon), EO858464, e.g. Org-37432 (Organon), WO9847876, e.g. Org-37476 (Organon), WO9807308, e.g. Org-39430 (Organon), EP217286, e.g. OS-396, CA2152205, e.g. S-30266 (Adir), EP792883, e.g. S-31214 and S-31922 (Servier), EP471651, e.g. SDZ-217766 and SDZ-MTH-958 (Novartis), WO9513274, e.g. UK-179094 (Pfizer), WO9716444, e.g. UK-285954 (Pfizer), WO9801428, e.g. XU-817 (BMS), JP96020597, US5510369, WO9736580, WO9847876, WO9847876, WO9746553, WO9842342, WO9746553, EP863755, US5891909, WO991516 EP0815103, US6117888, WO0075134, WO0075134, WO013832 EP0944590, WO0264140, EP1117660, EP0944590 and EP0944590.

As a further aspect of the present invention, there is provided the use of a tryptase inhibitor in the manufacture of a medicament for the treatment of a disease mediated by inhibition of a channel activating protease (CAP), e.g. a respiratory disease, most suitably cystic fibrosis or chronic obstructive pulmonary disease (COPD). Suitable mast cell tryptase inhibitors for use in the present invention include those compounds specifically and generally described in WO9420527, particularly APC-366 (Celera), and the compounds APC-2059 (Bayer), AVE-8923 (Sanofi-Aventis), MOL-6131 (Molecumetics) and M-58539 (Mochida).

As a further aspect of the present invention, there is provided the use of a kallikrein inhibitor in the manufacture of a medicament for the treatment of a disease mediated by inhibition of a channel activating protease (CAP), e.g. a respiratory disease, most suitably cystic fibrosis or chronic obstructive pulmonary disease (COPD). Suitable kallikrein inhibitors for use in the present invention include cetraxate and ecallantide.

As a further aspect of the present invention, there is provided the use of a trypsin inhibitor in the manufacture of a medicament for the treatment of a disease mediated by inhibition of a channel activating protease (CAP), e.g. a respiratory disease, most suitably cystic fibrosis or chronic obstructive pulmonary disease (COPD). Suitable trypsin inhibitors for use in the present invention include patamostat mesylate and those compounds generally or specifically described in US6469036, e.g. RWJ-58643 (J&J), EP556024, e.g. TO-195 (Torii), US6469036, e.g. RWJ-56423 (Ortho-McNeil), JP96020570, e.g. TT-S24 (Teikoko Chemical), EP588655 and WO0181314.

The serine protease inhibitor of the present invention is preferably a trypsin-like serine protease inhibitor such as a trypsin, matriptase or prostasin (PRSS8) inhibitor.

Prostasin is a trypsin-like serine protease that is present in a variety of mammalian tissues. It is a membrane anchored protease that is expressed on the extra-cellular membrane of cells but that can also be secreted into body fluids such as semen, urine and airway surface liquid. Prostasin, together with proteases such as matriptase, mCAP2, mCAP3, trypsin and neutrophil elastase, can stimulate the activity of the amiloride-sensitive epithelial sodium channel (ENaC). Inhibiting these enzymes can induce changes in epithelial ion transport and therefore fluid homeostasis across epithelial membranes. For example, CAP inhibition in the kidney is thought to promote diuresis, whilst CAP inhibition in the airways promotes the clearance of mucus and sputum in lung. CAP inhibition in the kidney may therefore be used therapeutically to treat hypertension. CAP inhibition in the airways prevents the stagnation of respiratory secretions that otherwise tends to make sufferers vulnerable to secondary bacterial infections.

Channel activating protease inhibitors are compounds that inhibit the activity of proteases that stimulate the activity of ion channels, especially the epithelial sodium channel. As a further aspect of the present invention, there is provided the use of channel activating protease inhibitors which is a serine protease inhibitor such as antipain, aprotinin, benzamidine, camostat, gabexate, leupeptin, nafamostat, pepstatin A, ribavirin, sepimostat and ulinastatin, for use in the treatment of a respiratory disease, most suitably cystic fibrosis or COPD. Preferred serine protease inhibitors are synthetic trypsin-like serine protease inhibitors, especially camostat, gabexate, nafamostat and sepimostat.

The invention provides, in a further aspect, use of a compound of formula I 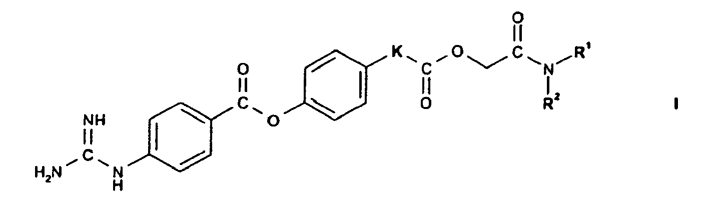 in free form or in the form of a pharmaceutically acceptable salt for the preparation of a medicament for the treatment of a disease mediated by inhibition of a channel activating protease, wherein R¹ and R², which may be the same or different, each represents hydrogen or C₁-C₃-alkyl; and K is a carbon-to-carbon covalent bond, a methylene group, an ethylene group or a vinylene group. "C₁-C₃-alkyl" as used herein denotes straight chain or branched alkyl that contains one to three carbon atoms.

Depending upon the nature of the variables and the corresponding number of centres of asymmetry and also upon the starting materials and procedures chosen, the compounds of formula I may be obtained in the form of mixtures of stereoisomers, for example mixtures of diastereoisomers or mixtures of enantiomers, such as racemates, or possibly also in the form of pure stereoisomers. Mixtures of diastereoisomers obtainable in accordance with the process or by some other method can be separated in customary manner into mixtures of enantiomers, for example racemates, or into individual diastereoisomers, for example on the basis of the physico-chemical differences between the constituents in known manner by fractional crystallisation, distillation and/or chromatography. Advantageously the more active isomer is isolated.

The compounds represented by formula I are capable of forming acid addition salts, particularly pharmaceutically acceptable acid addition salts. Pharmaceutically acceptable acid addition salts of the compound of formula I include those of inorganic acids, for example, hydrohalic acids such as hydrofluoric acid, hydrochloric acid, hydrobromic acid or hydroiodic acid, nitric acid, sulfuric acid, phosphoric acid; and organic acids, for example aliphatic monocarboxylic acids such as formic acid, acetic acid, trifluoroacetic acid, propionic acid and butyric acid, aliphatic hydroxy acids such as lactic acid, citric acid, tartaric acid or malic acid, dicarboxylic acids such as maleic acid or succinic acid, aromatic carboxylic acids such as benzoic acid, p-chlorobenzoic acid, diphenylacetic acid, *para-*biphenyl benzoic acid or triphenylacetic acid, aromatic hydroxy acids such as o-hydroxy-benzoic acid, p-hydroxy-benzoic acid, 1-hydroxynaphthalene-2-carboxylic acid or 3-hydroxy-naphthalene-2-carboxylic acid, cinnamic acids such as 3-(2-naphthalenyl)propenoic acid, *para*-methoxy cinnamic acid or *para*-methyl cinnamic acid, and sulfonic acids such as methanesulfonic acid or benzenesulfonic acid. These salts may be prepared from compounds of formula I by known salt-forming procedures. Preferred acid addition salts include salts with hydrochloric, sulfuric, phosphoric, hydrobromic, nitric, acetic, lactic, oxalic, maleic, fumaric, malic, tartaric, citric, ascorbic, benzenesulfonic, toluenesulfonic or methanesulfonic acid.

Compounds of formula I in free or pharmaceutically acceptable salt form are prepared using the method described in United States patent specification US 4,021,472, the contents of which is incorporated herein by reference.

Compounds of formula I suitable for use in the present invention include: N,N-Dimethylcarbamoylmethyl-p-hydroxybenzoate;
N-Methylcarbamoylmethyl-p-hydroxybenzoate;
N,N-Di-n-propylcarbamoylmethyl-p-hydroxybenzoate;
N,N-Dimethylcarbamoylmethyl-p-hydroxyphenylacetate;
N,N-Dimethylcarbamoylmethyl-p-hydroxycinnamate;
N,N-Dimethylcarbamoylmethyl-p-hydroxyphenylpropionate;
N-Methylcarbamoylmethyl-p-hydroxyphenylacetate;
Carbamoylmethyl-p-(p-guanidinobenzoyloxy)benzoate (especially the mesylate salt); N-Methylcarbamoyimethyl-p-(p-guanidinobenzoyloxy)benzoate (especially the mesylate salt);
N,N-Di-n-Propylcarbamoylmethyl-p-(p-guanidinobenzoyloxy)benzoate (especially the tosylate salt);
N,N-Dimethylcarbamoylmethyl-p-(p- guanidinobenzoyloxy)phenylacetate (especially the mesylate salt);
N,N-Dimethylcarbamoylmethyl-p-(p-guanidinobenzoyloxy)-cinnamate (especially the mesylate salt);
N,N-Dimethylcarbamoylmethyl-p-(p- guanidine-benzoyloxy)phenylpropionate (especially the mesylate salt); and
N-Methylcarbamoylmethyl-p-(p-guanidinobenzoyloxy)phenylacetate (especially the mesylate salt).

An especially preferred compound of formula I is N,N-dimethyl-carbamoylmethyl-p-(p-guanidinobenzoyloxy)phenylacetate, which has the non-proprietary name camostat. Camostat mesylate (Foipan^{™}) is a particularly preferred form of camostat and is a known trypsin-like serine protease inhibitor that has been used to treat acute symptoms of chronic pancreatitis. This compound is prepared using the method described in Example 13 of United States patent specification US 4,021,472.

As a further aspect of the present invention, there is provided a salt form of camostat, particularly for use in the present invention, preferably in the treatment of a respiratory disease, particularly cystic fibrosis or COPD, where the salt form is obtained from an acid selected from acetic, adipic, galactaric, glutaric, glycolic, hippuric, phosphoric, succinic, tartaric, stearic and 1-hydroxy-2-naphthoic acid, or a solvate, particularly a hydrate form thereof. A preferred salt of camostat, according to a yet further aspect of the invention, is selected from camostat hydrogensuccinate, camostat succinate, camostat phosphate, camostat acetate, camostat hydrogentartrate hemihydrate, camostat glycolate, camostat glycolate hemihydrate, camostat hippurate, camostat 1-hydroxy-2-naphthoate (xinafoate), camostat adipate and camostat glutarate. The preferred salts have different crystalline forms and physicochemical properties such as melting point, morphology etc., which may exhibit superior properties, e.g. solubility, bioavailability, stability and handling. The salts may also exhibit a lower propensity for irritation at the site of action. As a further aspect, there is provided the use of the afore-mentioned camostat salts in the uses, combinations and formulations of the present invention.

As a yet further aspect of the present invention, the inventors have discovered a new form of camostat free base (so-called Form II), characterised in the Examples hereinafter, formed by equilibrating camostat in ethanol/ water (1/1) at 25°C. The new form of camostat may exhibit superior properties, e.g. solubility, bioavailability, stability and handling. Thus, there is provided the use of camostat base form II in the uses, combinations and formulations of the present invention.

The invention provides, in a further aspect, a method of treating a disease mediated by inhibition of a channel activating protease in a subject, particularly a human subject, in need of such treatment, which comprises administering to said subject an effective amount of a serine protease inhibitor, especially a compound of formula I as hereinbefore defined. The serine protease inhibitor is preferably a synthetic trypsin-like serine protease inhibitor, especially a prostasin inhibitor such as camostat, or a suitable salt thereof.

Treatment of diseases mediated by inhibition of a channel activating protease in accordance with the invention may be prophylactic or symptomatic.

According to one aspect of the present invention, the compounds, salts and formulations of the invention may be used in the treatment of diseases mediated by inhibition of a channel activating protease, especially by a trypsin-like serine protease, such as prostasin. Such diseases include diseases associated with the regulation of fluid volumes across epithelial membranes. For example, the volume of airway surface liquid is a key regulator of mucociliary clearance and the maintenance of lung health. The inhibition of a channel activating protease will promote fluid accumulation on the mucosal side of the airway epithelium thereby promoting mucus clearance and preventing the accumulation of mucus and sputum in respiratory tissues (including lung airways). Such diseases include respiratory diseases such as cystic fibrosis, primary ciliary dyskinesia, chronic bronchitis, chronic obstructive pulmonary disease (COPD), asthma, respiratory tract infections (acute and chronic; viral and bacterial, e.g. cough and the common cold) and lung carcinoma. Diseases mediated by inhibition of channel activating proteases also include diseases other than respiratory diseases that are associated with abnormal fluid regulation across an epithelium, perhaps involving abnormal physiology of the protective surface liquids on their surface, for example xerostomia (dry mouth) or keratoconjunctivitis sire (dry eye). Furthermore, CAP regulation of ENaC in the kidney could be used to promote diuresis and thereby induce a hypotensive effect. The compounds for use in the present invention may also be useful in the treatment of hypertension, heart failure and diseases associated with hypertension or heart failure.

The compounds for use in the present invention may also be useful in the treatment of upper respiratory tract diseases such as sinusitis or allergic rhinitis.

Chronic obstructive pulmonary disease includes chronic bronchitis or dyspnea associated therewith, emphysema, as well as exacerbation of airways hyperreactivity consequent to other drug therapy, in particular other inhaled drug therapy. The invention is also applicable to the treatment of bronchitis of whatever type or genesis including, e.g., acute, arachidic, catarrhal, croupus, chronic or phthinoid bronchitis.

Asthma includes both intrinsic (non-allergic) asthma and extrinsic (allergic) asthma, mild asthma, moderate asthma, severe asthma, bronchitic asthma, exercise-induced asthma, occupational asthma and asthma induced following bacterial infection. Treatment of asthma is also to be understood as embracing treatment of subjects, e.g. of less than 4 or 5 years of age, exhibiting wheezing symptoms and diagnosed or diagnosable as "wheezy infants", an established patient category of major medical concern and now often identified as incipient or early-phase asthmatics. (For convenience this particular asthmatic condition is referred to as "wheezy-infant syndrome".)

The suitability of a channel activating protease inhibitor, such as a prostasin inhibitor, for the treatment of a disease mediated by inhibition of a channel activating protease, may be tested by determining the inhibitory effect of the channel activating protease inhibitor on:
(1) the native, isolated, purified or recombinant channel activating protease using a suitable biochemical assay format using the method described in Shipway et al. Biochemical and Biophysical Research Communications 2004; 324(2):953-63), and/or (2) the ion channel / ion transport function in suitable isolated cells or confluent epithelia using the methods described in Bridges et al., American Journal of Physiology Lung Cell Molecular Physiology 2001;281(1):L16-23) and Donaldson et al., Journal of Biological Chemistry 2002;277(10):8338-45).

Channel activating protease inhibitors, including the compounds of formula I and especially camostat or a pharmaceutically acceptable salt thereof, such as mesylate or the crystalline salt forms mentioned hereinbefore, are also useful as co-therapeutic agents for use in combination with other drug substances such as anti-inflammatory, bronchodilatory, antihistamine or anti-tussive drug substances, particularly in the treatment of cystic fibrosis or obstructive or inflammatory airways diseases such as those mentioned hereinbefore, for example as potentiators of therapeutic activity of such drugs or as a means of reducing required dosaging or potential side effects of such drugs.

A compound for use in the present invention may be mixed with the other drug substance in a fixed pharmaceutical composition or it may be administered separately, before, simultaneously with or after the other drug substance.

Accordingly the invention includes a combination of channel activating protease inhibitor, preferably camostat or a suitable salt thereof with an anti-inflammatory, bronchodilatory, antihistamine, anti-tussive, antibiotic or DNase drug substance, said channel activating protease inhibitor and said drug substance being in the same or different pharmaceutical composition.

Suitable anti-inflammatory drugs include steroids, in particular glucocorticosteroids such as budesonide, beclamethasone dipropionate, fluticasone propionate, ciclesonide or mometasone furoate, or steroids described in international patent application WO 02/88167, WO 02/12266, WO 02/100879, WO 02/00679 (especially those of Examples 3, 11, 14, 17, 19, 26, 34, 37, 39, 51, 60, 67, 72, 73, 90, 99 and 101), WO 03/35668, WO 03/48181, WO 03/62259, WO 03/64445, WO 03/72592, WO 04/39827 and WO 04/66920; non-steroidal glucocorticoid receptor agonists, such as those described in DE 10261874, WO 00/00531, WO 02/10143, WO 03/82280, WO 03/82787, WO 03/86294, WO 03/104195, WO 03/101932, WO 04/05229, WO 04/18429, WO 04/19935 and WO 04/26248; LTD4 antagonists such as montelukast and zafirlukast; PDE4 inhibitors such cilomilast (Ariflo® GlaxoSmithKiine), Roflumilast (Byk Gulden),V-11294A (Napp), BAY19-8004 (Bayer), SCH-351591 (Schering-Plough), Arofylline (Almirall Prodesfarma), PD189659 / PD168787 (Parke-Davis), AWD-12-281 (Asta Medica), CDC-801 (Celgene), SelCID(TM) CC-10004 (Celgene), VM554/UM565 (Vemalis), T-440 (Tanabe), KW-4490 (Kyowa Hakko Kogyo), and those disclosed in WO 92/19594, WO 93/19749, WO 93/19750, WO 93/19751, WO 98/18796, WO 99/16766, WO 01/13953, WO 03/104204, WO 03/104205, WO 03/39544, WO 04/000814, WO 04/000839, WO 04/005258, WO 04/018450, WO 04/018451, WO 04/018457, WO 04/018465, WO 04/018431, WO 04/018449, WO 04/018450, WO 04/018451, WO 04/018457, WO 04/018465, WO 04/019944, WO 04/019945, WO 04/045607 and WO 04/037805; and adenosine A_{2B} receptor antagonists such as those described in WO 02/42298.

Suitable bronchodilatory drugs include beta-2 adrenoceptor agonists such as albuterol (salbutamol), metaproterenol, terbutaline, salmeterol fenoterol, procaterol, and especially, formoterol, carmoterol and pharmaceutically acceptable salts thereof, and compounds (in free or salt or solvate form) of formula I of WO 00/75114, which document is incorporated herein by reference, preferably compounds of the Examples thereof, especially a compound (5-[(R)-2-(5,6-Diethyl-indan-2-ylamino)-1-hydroxy-ethyl]-8-hydroxy-1H-quinolin-2-one) and pharmaceutically acceptable salts thereof, as well as compounds (in free or salt or solvate form) of formula I of WO 04/16601, and also compounds of EP 1440966, JP 05025045, WO 93/18007, WO 99/64035, US 2002/0055651, WO 01/42193, WO 01/83462, WO 02/66422, WO 02/ 70490, WO 02/76933, WO 03/24439, WO 03/42160, WO 03/42164, WO 03/72539, WO 03/91204, WO 03/99764, WO 04/16578, WO 04/22547, WO 04/32921, WO 04/33412, WO 04/37768, WO 04/37773, WO 04/37807, WO 04/39762, WO 04/39766, WO 04/45618 WO 04/46083 and WO 04/80964.

Suitable bronchodilatory drugs also include anticholinergic or antimuscarinic agents, in particular ipratropium bromide, oxitropium bromide, tiotropium salts and CHF 4226 (Chiesi), and glycopyrrolate, but also those described in EP 424021, US 3714357, US 5171744, WO 01/04118, WO 02/00652, WO 02/51841, WO 02/53564, WO 03/00840, WO 03/33495, WO 03/53966, WO 03/87094, WO 04/018422 and WO 04/05285.

Suitable dual anti-inflammatory and bronchodilatory drugs include dual beta-2 adrenoceptor agonist / muscarinic antagonists such as those disclosed in US 2004/0167167, WO 04/74246 and WO 04/74812.

Suitable antihistamine drug substances include cetirizine hydrochloride, acetaminophen, clemastine fumarate, promethazine, loratidine, desloratidine, diphenhydramine and fexofenadine hydrochloride, activastine, astemizole, azelastine, ebastine, epinastine, mizolastine and tefenadine as well as those disclosed in JP 2004107299, WO 03/099807 and WO 04/026841.

Suitable antibiotics include macrolide antibiotics, for example tobramycin (TOBI™).

Suitable DNase drug substances include domase alfa (Pulmozyme™), a highly purified solution of recombinant human deoxyribonuclease I (rhDNase), which selectively cleaves DNA. Dornase alfa is used to treat cystic fibrosis.

Further suitable combinations with anti-inflammatory drugs are those with antagonists of chemokine receptors, e.g. CCR-1, CCR-2, CCR-3, CCR-4, CCR-5, CCR-6, CCR-7, CCR-8, CCR-9 and CCR10, CXCR1, CXCR2, CXCR3, CXCR4, CXCR5, particularly CCR-5 antagonists such as Schering-Plough antagonists SC-351125, SCH-55700 and SCH-D, Takeda antagonists such as N-[[4-[[[6,7-dihydro-2-(4-methyl-phenyl)-5H-benzo-cyclohepten-8-yl]carbonyl]amino]phenyl]-methyl]tetrahydro-N,N-dimethyl-2H-pyran-4-amin-ium chloride (TAK-770), and CCR-5 antagonists described in US 6166037 (particularly claims 18 and 19), WO 00/66558 (particularly claim 8), WO 00/66559 (particularly claim 9), WO 04/018425 and WO 04/026873.

Camostat and suitable salts and the compounds, salts and formulations for use in the present invention may also be combined with inhaled osmolytes, such as hypertonic saline, mannitol or dextran.

The compounds for use in the present invention may also be combined with P2Y2 antagonists or agonists, CLC2 activators, ENaC inhibitors or PDE-5 inhibitors.

The compounds, salts and formulations for use in the present invention may also be combined with an NSAID, such as ibuprofen.

In the treatment of a disease mediated by inhibition of prostasin in accordance with the invention, a channel activating protease inhibitor, including a compound I, particularly camostat, in free form or in pharmaceutically acceptable salt form, may be administered by any appropriate route, for example orally, e.g. in tablet, capsule or liquid form, parenterally, for example in the form of an injectable solution or suspension, or intranasally, for example in the form of an aerosol or other atomisable formulation using an appropriate intranasal delivery device, e.g. a nasal spray such as those known in the art, or by inhalation, which is preferred, especially for use with a nebulizer.

The channel activating protease inhibitor may be administered in a pharmaceutical composition together with a pharmaceutically acceptable diluent or carrier. Such compositions may be, for example dry powders, tablets, capsules and liquids, but also injection solutions, infusion solutions or inhalation powders, aqueous and propellant based, solutions or suspensions, which may be prepared using other formulating ingredients and techniques known in the art.

The dosage of the channel activating protease inhibitor in free form or in pharmaceutically acceptable salt form can depend on various factors, such as the activity and duration of action of the active ingredient, the severity of the condition to be treated, the mode of administration, the species, sex, ethnic origin, age and weight of the subject and/or its individual condition. In a normal case the daily dose for administration, for example oral administration, to a warm-blooded animal, particularly a human being, weighing about 75 kg is estimated to be from approximately 0.7 mg to approximately 1400 mg, especially from approximately 5 mg to approximately 200 mg. That dose may be administered, for example, in a single dose or in several part doses of, for example, from 5 to 200 mg.

When the composition comprises an aerosol formulation, it preferably contains, for example, a hydro-fluoro-alkane (HFA) propellant such as HFA134a or HFA227 or a mixture of these, and may contain one or more co-solvents known in the art such as ethanol (up to 20% by weight), and/or one or more surfactants such as oleic acid or sorbitan trioleate, and/or one or more bulking agents such as lactose. When the composition comprises a dry powder formulation, it preferably contains, for example, the channel activating protease inhibitor having a particle diameter up to 10 microns, optionally together with a diluent or carrier, such as lactose, of the desired particle size distribution and a compound that helps to protect against product performance deterioration due to moisture e.g. magnesium stearate. When the composition comprises a nebulised formulation, it preferably contains, for example, the channel activating protease inhibitor either dissolved, or suspended, in a vehicle containing water, a co-solvent such as ethanol or propylene glycol and a stabiliser, which may be a surfactant.

The invention includes (A) a compound of formula I, especially camostat, or a pharmaceutically acceptable salt thereof, in inhalable form, e.g. in an aerosol or other atomisable composition or in inhalable particulate, e.g. micronised, form, (B) an inhalable medicament comprising a compound of formula I in inhalable form; (C) a pharmaceutical product comprising a compound of formula I in inhalable form in association with an inhalation device; and (D) an inhalation device containing a compound of formula I in inhalable form.

In another embodiment, the inhalation route of administration is in a powder form. The active ingredient may be used as a powder with a particle size of 0.5 to 10 micrometers, preferably 0.5-5 micrometers which can be produced by a variety of conventional techniques, such as jet-milling, spray-drying, solvent precipitation, and the like. One widely used formulation approach is to mix the fine active drug particles with a coarse bulking powder with a particle size of 10 to 500µm. The bulking powder is selected from saccharides, preferably lactose, sucrose, glucose, galactose, fructose, trehalose and raffinose, most preferably lactose. Preferably the particle size of the finely-divided solid powder should for physiological reasons be less than 25 microns and preferably less than about 10 microns in diameter. The particle size of the powder for inhalation therapy should most preferably be in the range of 2 to 10 microns.

Other embodiments of the present the invention include aerosol formulations which comprise the active ingredient suspended or dissolved in a suitable aerosol propellant, such as a chlorofluorocarbon (CFC) or a hydrofluorocarbon (HFC). Suitable CFC propellants include trichloromonofluoromethane (propellant 11), dichlorotetrafluoromethane (propellant 114), and dichlorodifluoromethane (propellant 12). Suitable HFC propellants include tetrafluoroethane (HFC-134a) and heptafluoropropane (HFC-227). The propellant comprises 40 to 90% by weight of the total inhalation composition. A yet further suitable propellant is ethanol.

Preferably, for incorporation into the aerosol propellant, the active ingredient, e.g. camostat or a pharmaceutically acceptable salt thereof, will be processed into respirable particles as described for the dry powder formulations. The particles are then suspended in the propellant, typically being coated with a surfactant to enhance their dispersion properties. Such surfactants include sorbitan tiroleate, oleyl alcohol, oleic acid, lecithin or oils derived from natural sources, such as, corn oil, olive oil, cotton seed oil and sunflower seed oil are useful in keeping the suspended particles form agglomerating.

As a further aspect of the invention, a compound for use in the present invention may be prepared as a formulation suitable for nebulisation. Such formulations contain excipients physiologically compatible with the bronchial epithelium, usually consisting of co-solvents, preservatives, chelating agents, pH and tonicity regulators and surfactants. Suitable excipients include but are not limited to, propylene glycol, ethanol, glycerin, benzalkonium chloride, sodium edentate, ascorbic acid, citric acid, hydrochloric acid, sodium hydroxide, sodium chloride, oleic acid, and lecithin. The nebulised formulation may be prepared by standard manufacturing techniques.

As a further aspect of the invention, a compound for use in the present invention, preferably camostat or a pharmaceutically acceptable salt thereof, may be prepared in a lyophilised form for subsequent reconstitution in a physiologically acceptable vehicle for inhalation immediately prior administration. The lyophilised formulation may include the following optional additional excipients, lactose, mannitol and glucose. The lyophilised product may be prepared by a aseptic process first involving dissolving the active ingredient and excipients in an aqueous vehicle. The resulting solution is then filled in glass vials and subsequently freeze dried. The lyophilized camostat suitably includes lactose, suitably in a ratio of camostat or a salt form:lactose of 1:1 to 1:10.

As a further aspect of the invention, a formulation for use in the present invention, preferably a nebulized formulation of camostat or a pharmaceutically acceptable salt thereof, may be taste-masked by various approaches including addition of suitable further excipients either in the solution formulation or as an ingredient in the lyophilized form or vehicle for reconstitution. Suitable excipients for taste-masking according to the invention include but not limited to, saccharine sodium, aspartame, menthol and polyalcohols such as sorbitol and xylitol. The taste-masked formulation may be prepared by known and standard techniques, e.g. according to the methods described in W02005/037246 and Manfred Keller, Antonio Manuel Fernandes Raposo et al. "Taste masking of a pentoxifylline formulation for pulmonary application (patent application). The taste-masked formulation of camostat suitably comprises sachharine sodium, suitably in a camostat or salt form: saccharine sodium ratio of 100:1 to 1:5.

### Biological Examples

The channel activating protease inhibitor, camostat mesylate, inhibits prostasin (human and guinea pig) and matriptase (human), attenuates the amiloride-sensitive, ENaC-mediated short circuit current in cultured human bronchial epithelial cells, and attenuates the tracheal potential difference in the guinea pig

***Biochemical assays:*** Recombinant human prostasin and matriptase and guinea pig prostasin are generated according to methods described in Shipway et al., Biochemical and Biophysical Research Communications 2004; 324(2):953-63). The recombinant enzymes are incubated in an electrolyte buffer containing the test compounds or vehicle in a suitable multiple well assay plate such as a 96 or 384 well plate. At a defined time after the mixing of enzyme with compound or vehicle, a suitable fluorescent peptide substrate is added to the assay mixture. As substrate becomes cleaved by the active enzyme, fluorescence (measured using a suitable fluorescence plate reader) increases and the rate of turnover of substrate (i.e. enzyme activity) can be quantified and thus the inhibitory effect of any test compound. The efficacy of test compounds is expressed as the concentration that induces a 50% attenuation in the enzyme activity (Kᵢ).

***Epithelial ion transport:*** Human bronchial epithelial cells are cultured according to methods described in Danahay et al., American Journal of Physiology Lung Cell Molecular Physiology 2002; 282(2):L226-36). When suitably differentiated (days 14-21 after establishing an apical-air interface) epithelial cells are treated with either vehicle, aprotinin (200 µg/ml) or test compound for 90 minutes. Epithelia are then placed into Ussing Chambers as described in Danahay et al., American Journal of Physiology Lung Cell Molecular Physiology 2002; 282(2):L226-36) maintaining the concentration of vehicle, aprotinin or test compound on the apical side of the epithelia. Short circuit current (ISC) is then measured by voltage clamping the epithelia to zero millivolts. The amiloride-sensitive ISC is then measured by the addition of amiloride (10 µM) to the apical surface of the epithelia. The potency of the test compound is expressed as the concentration inducing a 50% inhibition of the total aprotinin-sensitive component of the amiloride-sensitive ISC.

***Tracheal potential difference (in vivo):*** Guinea pigs are anaesthetized using a short acting inhalation anaesthesia such as halothane and N₂O. Whilst under short acting anaesthesia an oral gavage needle is inserted into the trachea via the oropharangeal route. Once inside the trachea, a small volume (50-200 µl) of vehicle or test compound, in a suitable aqueous-based diluent, is instilled into the airways. Animals then recover and become fully ambulatory. Alternatively test compounds can be administered to animals using aerosol or dry powder dosing. At a defined time after dosing, the animals are surgically anaesthetized using a suitable anaesthesia such as ketamine and xylazine. The trachea is then exposed and a plastic agar bridge electrode is inserted into the tracheal lumen. A reference electrode is also inserted into the layers of muscle in the animal's neck. The tracheal potential difference is then measured using a suitable high impedance voltmeter as described in Takahashi et al., Toxicol Appl Pharmacol. 1995; 131(1):31-6. The potency of the test compound is expressed as the dose inducing a 50% reduction in the sensitive-component of the tracheal potential difference.

The results are shown in the tables 1 and 2 below:

**Table 1 Summary of in vitro & in vivo data with Camostat mesylate**

| rh-prostasin (Ki) | rh-matriptase (Ki) | rGPig-prostasin (Ki) | HBEC (IC₅₀) | GPig tracheal PD in vivo (ED₅₀) |
|---|---|---|---|---|
| 0.429 µM | 0.005 µM | 0.098 µM | 0.05 µM | 2 hr = 3.3 µg/kg (i.t.) |
| | | | | 5 hr = 26 µg/kg (i.t.) |

**Table 2 Effects of Camostat mesylate on Guinea pig tracheal potential difference in vivo**

| | Camostat mesylate dose (µg/kg) by intra-tracheal administration | | | | | | | |
|---|---|---|---|---|---|---|---|---|
| | 0 | 1 | 3 | 10 | 30 | 100 | 300 | 1000 |
| 2 hr | -10.3 | -10.0 | -7.3 | -6.4 | -5.3 | -4.7 | | |
| | ±0.6 | ±0.7 | ±0.5 | ±0.5 | ±0.3 | ±0.5 | | |
| 5 hr | -9.9 | | -10.8 | -9.7 | -7.5 | -6.5 | -5.2 | -5.7 |
| | ±0.6 | | ±1.0 | ±1.5 | ±0.4 | ±0.3 | ±0.3 | ±0.4 |

Mean absolute values of the tracheal potential difference (± s.e.mean) are shown at either 2 or 5 hours after intra-tracheal dosing with vehicle or camostat mesylate.

### Examples of salt forms of camostat

X-ray powder diffraction patterns were measured using a Bruker STOE instrument with CuK alpha radiation source.
FT-IR spectrum were recorded in Nujol mull between 2 KBr plates using a Bruker IFS-55.

### Example 1 Hydrogensuccinate Form A

A suspension of 5.0 g camostat base (12.55 mmoles) and 1.48 g succinic acid (12.55 mmoles) in 50 ml ethanol 90% is heated to ca. 65°C. 10 ml water are then added dropwise at 65-70°C. The resulting clear solution is allowed to cool. Some seeds are added at 50°C and crystallization takes place slowly. The suspension is stirred for 17 hours at room temperature and then filtered. The crystals are washed with 30 ml ethanol 90% and dried at 60°C and ca. 10 mbar for 20 hours.
Yield: 5.70 g white powder (87.9%)
Purity HPLC: 99.5% (a) Water assay: <0.2 % m/m Elemental analysis:
Calc.: 55.81% C; 5.46% H; 10.85% N; 27.88% O
Found: 55.73% C; 5.40% H; 10.96% N; 28.04% O
The XRPD for the hydrogensuccinate is summarized in Table 3.

**Table 3. Powder X-Ray Diffraction Peaks - hydrogensuccinate**

| 2-theta (deg) | d-spacings (Å) | Relative intensity |
|---|---|---|
| 11.7 | 7.55 | Medium |
| 14.2 | 6.21 | Strong |
| 16.2 | 5.44 | Low |
| 16.5 | 5.36 | Low |
| 17.0 | 5.20 | Medium |
| 18.7 | 4.73 | Low |
| 19.5 | 4.53 | Medium |
| 21.6 | 4.09 | Low |
| 22.8 | 3.88 | Medium |
| 22.9 | 3.87 | Strong |
| 23.1 | 3.83 | Medium |
| 24.3 | 3.64 | Medium |
| 24.6 | 3.61 | Low |
| 25.7 | 3.45 | Low |
| 26.9 | 3.30 | Low |
| 30.5 | 2.92 | Strong |

FT-IR Main IR bands: 3381; 3119; 1744; 1729; 1512; 1729; 1512; 1463; 1270; 1220; 1182; 1158; 1078; 1020; 760; 704 cm⁻¹

### Example 2 Succinate Form A

A suspension of 3.0 g camostat base (7.53 mmoles) in 30 ml ethanol 90% is heated to 75°C. A solution of 0.45 g succinic acid (3.76 mmoles) in 3 ml water is then added dropwise and the dropping funnel is rinsed with 6 ml ethanol 90%. The clear solution is allowed to cool. Seeds addition at 60°C is followed by crystallization. The slurry is stirred over night at room temperature and then filtered by suction. The residue is washed with 30 ml ethanol 90% and dried at 60°C / ca 10 mbar for 20 hours.
Yield: 3.03 g white crystals (88.0%)
HPLC purity: 98.0% (a)
Water assay: < 0.3 % m/m
Elemental analysis:
Calc.: 57.76 % C; 5.51 % H; 12.25 % N; 24.48 % O
Found: 57.55 % C; 5.64 % H; 12.22 % N; 24.60 % O
The XRPD for the succinate is summarized in Table 4. The XRPD shows a strong diffraction peak at 17.5°.

**Table 4. Powder X-Ray Diffraction Peaks - Succinate**

| 2-theta (deg) | d-spacings (Å) | Relative intensity |
|---|---|---|
| 2.4 | 36.57 | S |
| 8.3 | 10.52 | M |
| 12.3 | 7.15 | S |
| 13.2 | 6.68 | M |
| 14.3 | 6.18 | L |
| 14.6 | 6.05 | S |
| 15.8 | 5.58 | M |
| 16.6 | 5.32 | M |
| 17.1 | 5.15 | M |
| 17.5 | 5.04 | S |
| 18.1 | 4.89 | L |
| 18.5 | 4.76 | S |
| 19.3 | 4.58 | L |
| 20.3 | 4.35 | M |
| 21.5 | 4.12 | L |
| 23.1 | 3.83 | S |
| 23.8 | 3.73 | M |
| 24.3 | 3.64 | L |
| 26.3 | 3.38 | M |

FT-IR Main IR hands: 3306; 1747; 1727; 1651; 1586; 1555; 1514; 1465; 1422; 1266; 1206; 1173; 1143; 1074; 878; 853, 743; 694 cm⁻¹

### Example 3 Phosphate Form A (2 examples with identical XRPD diagram)

### 3.1 Phosphate (crystallized from ethanol / water)

A solution of 1,45 g phosphoric acid 85% (12.55 mmoles) in 1 ml water is added dropwise to a suspension of 5. 0 g camostat base (12.55 mmoles) in 99 ml ethanol at room temperature. The mixture is heated at 75°C and 40 ml water are added over ca. 5 min. The resulting clear solution is allowed to cool. Crystallization takes place after seeding at 55°C. The suspension is stirred over night at r. t. and filtered. The solid is washed first with 30 ml ethanol 70 % and then with 10 ml acetone. The crystals are dried at 60°C and ca. 10 mbar for 20 h. Yield: 4.70 g white powder (75.4 %)
HPLC purity: 99. 8% (a) Water assay: <0.2% m/m
Elemental analysis:
Calc.: 48.39 % C; 5.08 % H; 11.29 % N; 29.01 % O 6.24 % P
Found: 48.28 % C; 5.02 % H; 11.32 % N; 28.83 % O 6.01 % P

### 3.2 Phosphate Form A (crystallized from water)

A solution of 1,45 g phosphoric acid 85% (12.55 mmoles) in 5 ml water is added dropwise to a suspension of 5. 0 g camostat base (12.55 mmoles) in 45 ml water at 50°C. The resulting clear solution is allowed to cool. Crystallization takes rapidly place after seeding at 40°C. The very thick suspension is diluted with 20 ml water and stirred over night at r. t. After filtration the solid is washed first with 20 ml water and then with 10 ml acetone. The crystals are dried at 60°C and ca. 10 mbar for 20 h.
Yield: 4.59 g white powder (73.7 %)
HPLC purity: 99. 7% (a) Water assay: <0.2% m/m
Elemental analysis:
Calc.: 48.39 % C; 5.08 % H; 11.29 % N; 29.01 % O; 6.24 % P
Found: 48.34 % C; 4.95 % H; 11.28 % N; 29.16 % O; 5.92 % P

The XRPD for the phosphate is summarized in Table 5. The XRPD shows a strong diffraction peak at 16.8°.

**Table 5. Powder X-Ray Diffraction Peaks - Phosphate**

| 2-theta (deg) | d-spacings (Å) | Relative intensity |
|---|---|---|
| 3.7 | 23.77 | Strong |
| 12.1 | 7.25 | Low |
| 15.0 | 5.86 | Low |
| 16.8 | 5.27 | Strong |
| 17.9 | 4.95 | Low |
| 18.2 | 4.85 | Low |
| 19.9 | 4.44 | Low |
| 20.6 | 4.29 | Medium |
| 21.0 | 4.22 | Strong |
| 21.7 | 4.07 | Strong |
| 22.1 | 4.01 | Medium |
| 22.3 | 3.96 | Low |
| 23.7 | 3.73 | Low |
| 24.0 | 3.70 | Strong |
| 24.5 | 3.62 | Low |
| 24.8 | 3.57 | Low |
| 27.1 | 3.28 | Low |

IR- Main IR bands: 3412; 3230; 1746; 1729; 1683; 1648; 1610; 1570; 1513; 1463; 1334; 1272; 1218, 1202; 1021; 940 cm⁻¹

### Example 4 Acetate Form A

0.457 g acetic acid (7.53 mmoles) are added to a suspension of 3.0 g camostat base (7.53 mmoles) in 28 ml isopropanol and 2 ml water at room temperature. The resulting solution is heated to 45°. Crystallization takes then place spontaneously at 45°C. After cooling, the slurry is stirred at 25°C for 18 h. The salt is collected through filtration and washed first with 30 ml Isopropanol / water 9 / 1 and then with 14 ml isopropanol. The crystals are dried at 80°C and ca. 10 mbar for 20 h.
Yield: 3.09 g white powder (89.5 %)
HPLC purity: 99.5 % (a) Water assay: <0.3%
Elemental analysis:
Calc.: 57.64 % C; 5.72 % H; 12.22 % N; 24.43 % O
Found: 57.49 % C; 5.83 % H; 12.08 % N; 24.48 % O
The XRPD for the acetate is summarized in Table 6. The XRPD shows a strong diffraction peak at 19.0°.

**Table 6. Powder X-Ray Diffraction Peaks - Acetate**

| 2-theta (deg) | d-spacings (Å) | Relative intensity |
|---|---|---|
| 3.6 | 24.50 | L |
| 7.1 | 12.27 | L |
| 14.1 | 6.23 | L |
| 14.6 | 6.05 | S |
| 15.8 | 5.58 | L |
| 17.7 | 4.98 | L |
| 18.7 | 4.72 | M |
| 19.0 | 4.64 | S |
| 20.0 | 4.42 | S |
| 24.5 | 3.61 | L |
| 24.8 | 3.58 | M |
| 25.5 | 3.47 | M |
| 25.8 | 3.44 | S |
| 26.5 | 3.35 | M |
| 26.8 | 3.32 | L |
| 28.4 | 3.13 | L |
| 28.7 | 3.10 | L |
| 29.3 | 3.03 | L |

FT-IR Main IR bands: 3200; 1749; 1717; 1687; 1647; 1576; 1516; 1411; 1287; 1195; 1148; 1049; 1023; 887; 860; 653 cm⁻¹

### Example 5 Hydrogentartrate hemihydrate Form A

3.0 g camostat base (7.53 mmoles) and 1.13 g L (+) tartaric acid (7.53 mmoles) are suspended in 28 ml isopropanol und 2 ml water. The mixture is heated to 75°C. During heating initially sticky, semi solid constituents are transformed in a homogeneous suspension. The suspension is stirred at 75°C for 10 min. and then allowed to cool. The slurry becomes rapidly too thick. The suspension is gradually diluted with 6 ml water and 84 ml isopropanol over ca. 30 min. during cooling. After stirring over night at 25°C the crystallisate is filtered by suction and washed successively with a mixture of 27 ml isopropanol and 3 ml water and then with 30 ml isopropanol. The crystals are dried for 20 h at 80°C and ca. 10 mbar.
Yield: 4.16 g white powder (99.1 %)
HPLC purity: 99.2 % (a)
Water assay: 2.16 % m/m
Elemental analysis:
Calc.: 52.55 % C; 5.15 % H; 10.21 % N; 32.09 % O
Found: 51.73 % C; 5.36 % H; 10.09 % N; 33.01 % O
The XRPD for the hydrogentartrate hemihydrate is summarized in Table 7. The XRPD shows a strong diffraction peak at 13.3°.

**Table 7. Powder X-Ray Diffraction Peaks - Hydrogentartrate hemihydrate**

| 2-theta (deg) | d-spacings (Å) | Relative intensity |
|---|---|---|
| 5.4 | 16.11 | S |
| 9.4 | 9.35 | L |
| 12.6 | 6.99 | L |
| 13.3 | 6.63 | S |
| 15.3 | 5.75 | S |
| 16.3 | 5.42 | S |
| 16.6 | 5.30 | L |
| 18.1 | 4.88 | M |
| 18.8 | 4.69 | L |
| 19.7 | 4.49 | L |
| 21.8 | 4.06 | L |
| 22.5 | 3.93 | S |
| 24.0 | 3.69 | M |
| 26.0 | 3.41 | M |
| 26.5 | 3.35 | S |
| 18.1 | 3.16 | S |
| 28.4 | 3.13 | L |

FT-IR Main IR bands: 3404; 1721; 1660; 1566; 1518; 1462, 1377; 1281; 1202; 1183; 1169; 1143; 1083 cm⁻¹

### Example 6 Glycolate hemihydrate Form A

A suspension of 3.0 g camostat base (7.53 mmoles) and 0.58 g glycolic acid (7.53 mmoles) in 28 ml isopropanol and 2 ml water is slowly heated to ca. 45°C. A clear solution is first formed at 35°C and crystallization begins spontaneously at 45°C. The mixture is allowed to cool and the slurry stirred over night at r.t. The solid is isolated by filtration. The filter cake is washed first with 20 ml isopropanol / water = 9 / 1 (v / v) and then with 14 ml isopropanol. The salt is dried at 80°C and ca. 10 mbar for 20 h.
Yield: 3.70 g white powder (95.3 %)
Elemental analysis:
Calc.: 54.65% C; 5.63% H; 11.59% N; 28.13% O
Found: 54.54% C; 5.85% H; 11.40% N; 28.06% O
Water assay: 1.94 % (m/m)
HPLC purity: 99.4% (a)
The XRPD for the glycolate hemihydrate is summarized in Table 8. The XRPD shows a strong diffraction peak at 14.5° and 22.6°.

**Table 8. Powder X-Ray Diffraction Peaks - Glycolate hemihydrate**

| 2-theta (deg) | d-spacings (Å) | Relative intensity |
|---|---|---|
| 9.4 | 9.32 | L |
| 9.6 | 9.19 | L |
| 11.4 | 7.71 | L |
| 11.7 | 7.53 | M |
| 12.8 | 6.87 | M |
| 14.5 | 6.08 | S |
| 15.8 | 5.59 | L |
| 16.9 | 5.21 | L |
| 17.3 | 5.12 | M |
| 17.9 | 4.92 | L |
| 18.9 | 4.68 | S |
| 19.1 | 4.63 | L |
| 19.8 | 4.47 | M |
| 20.0 | 4.42 | L |
| 21.0 | 4.22 | M |
| 22.6 | 3.92 | S |
| 23.4 | 3.79 | L |

FT-IR Main IR bands: 3311; 1732; 1708; 1574; 1506; 1410; 1316; 1267; 1202; 1194; 1181; 1166; 1132; 1065; 1066; 1016763; 740 cm⁻¹

### Example 7 Glycolate Form A

A suspension of 3.0 g Camostat base (7.53 mmoles) in 50 ml ethanol is heated to 70 °C. A solution of 0.58 g glycolic acid (7.53 mmoles) in 4 ml ethanol is added dropwise and the dropping funnel is rinsed with 6 ml ethanol. The resulting clear solution is allowed to slowly cool. Crystallization takes spontaneously place at ca. 50°C. The white suspension is stirred at room temperature for 20 h and filtered. The crystals are washed with 30 ml ethanol and dried at 80°C and ca. 10 mbar for 20 h.
Yield: 3.44 g white powder (96.3 %)
Elemental analysis:
Calc.: 55.69 % C; 5.52 % H; 11.81 % N; 26.98 % O
Found: 55.53 % C; 5.74 % H; 11.76 % N; 27.04 % O
HPLC purity: 99.5% (a)
The XRPD for the glycolate is summarized in Table 9. The XRPD shows a strong diffraction peak at 19.9°.

**Table 9. Powder X-Ray Diffraction Peaks - Glycolate**

| 2-theta (deg) | d-spacings (Å) | Relative intensity |
|---|---|---|
| 9.3 | 9.49 | M |
| 10.5 | 8.35 | L |
| 11.1 | 7.94 | L |
| 13.6 | 6.49 | L |
| 14.1 | 6.25 | L |
| 17.5 | 5.04 | S |
| 19.9 | 4.45 | S |
| 20.5 | 4.31 | M |
| 21.1 | 4.20 | S |
| 22.5 | 3.94 | S |
| 22.8 | 3.88 | L |
| 23.4 | 3.79 | S |
| 24.4 | 3.64 | L |
| 26.4 | 3.36 | M |
| 27.8 | 3.20 | M |

FT-IR Main IR bands: 3345; 3070; 1729; 1669; 1636; 1582; 1515; 1410, 1317; 1285; 1265; 1208; 1129; 1069; 762 cm⁻¹

### Example 8 Xinafoate Form A

A suspension of 3.0 g camostat base (7.53 mmoles) in a mixture of 28 ml isopropanol and 2 ml water is heated to 60°C. A solution of 1.44 g 1-hydroxy-2-naphthoic acid (7.53 mmoles) in 27 ml isopropanol and 3 ml water is added at constant flow rate over ca 10 min. A clear solution is first observed and crystallization takes then rapidly place. The tick suspension is allowed to cool and gradually diluted with 27 ml isopropanol and 3 ml water. After stirring over night at 25°C the slurry is filtered. The solid is washed first with 10 ml isopropanol / water = 9 / 1 and then with 10 ml isopropanol. The product is dried first 2 h at 25 °C / vacuum and then 20 h at 80°C and ca. 10 mbar.
Yield: 4.38 g white powder (99.1 %)
Elemental analysis:
Calc.: 63.47 % C; 5.15 % H; 9.55 % N; 21.82 % O
Found: 62.89 % C; 5.25 % H; 9.37 % N; 22.28 % O
Water assay: 0.42 % (m/m) HPLC purity: 98.1% (a)
The XRPD for the xinafoate is summarized in Table 10. The XRPD shows a strong diffraction peak at 21.3°.

**Table 10. Powder X-Ray Diffraction Peaks - Xinafoate**

| 2-theta (deg) | d-spacings (Å) | Relative intensity |
|---|---|---|
| 6.9 | 12.77 | L |
| 10.0 | 8.80 | L |
| 11.1 | 7.93 | L |
| 12.3 | 7.18 | S |
| 14.3 | 6.18 | S |
| 16.3 | 5.42 | M |
| 16.5 | 5.34 | M |
| 17.0 | 5.20 | M |
| 17.4 | 5.07 | M |
| 18.0 | 4.91 | L |
| 18.7 | 4.72 | M |
| 20.5 | 4.32 | L |
| 21.3 | 4.16 | S |
| 24.1 | 3.68 | L |
| 24.9 | 3.56 | S |
| 25.7 | 3.46 | S |
| 26.0 | 3.41 | M |
| 27.1 | 3.27 | L |

FT-IR Main IR bands: 3440; 1753; 1738; 1514; 1464; 1438; 1401; 1306; 1260; 1206; 1172; 1140; 1075; 1014; 800; 779; 757 cm⁻¹

### Example 9 Hippurate Form A

A suspension of 3.0 g Camostat base (7.53 mmoles) in a mixture of 76.5 ml ethanol 95% and 8.5 ml water is heated to 65°C. 1.35 g hippuric acid (7.53 mmoles) are added. The resulting clear solution is allowed to cool and seeded at 50°C. The suspension is stirred over night at 25°C and filtered. The filter cake is washed with 30 ml ethanol 95% dried at 80°C and ca. 10 mbar for 20 h.
Yield: 3.63g white powder (83.4 %)
Elemental analysis:
Calc.: 60.31 % C; 5.41 % H; 12.13 % N; 22.16 % O
Found: 60.04 % C; 5.34 % H; 12.05 % N; 22.23 % O
Water assay: < 0.1 % (m/m) HPLC purity: 99.4%
The XRPD for the hippurate is summarized in Table 11. The XRPD shows a strong diffraction peak at 21.1°.

**Table 11. Powder X-Ray Diffraction Peaks - Hippurate**

| 2-theta (deg) | d-spacings (Å) | Relative intensity |
|---|---|---|
| 5.3 | 16.39 | M |
| 6.1 | 14.34 | M |
| 10.0 | 8.76 | L |
| 10.7 | 8.23 | L |
| 13.8 | 6.40 | M |
| 14.9 | 5.91 | L |
| 18.1 | 4.89 | M |
| 18.8 | 4.69 | M |
| 19.3 | 4.57 | M |
| 20.1 | 4.40 | S |
| 20.7 | 4.27 | L |
| 21.1 | 4.19 | S |
| 22.1 | 4.01 | S |
| 22.8 | 3.89 | M |
| 23.4 | 3.79 | M |
| 24.1 | 3.67 | L |
| 26.1 | 3.40 | L |
| 26.9 | 3.31 | M |
| 28.7 | 3.10 | M |

Main IR bands: 3378; 1731; 1718; 1656; 1572; 1513; 1458; 1415; 1380; 1276; 1202; 1178; 1168; 1145; 1080; 765; 720; 693; 673 cm⁻¹

### Example 10 Adipate Form A

A suspension of 3.0 g camostat base (7.53 mmoles) in 30 ml ethanol is heated at 65°C. Then a solution of 1.10 g adipic acid (7.53 mmoles) in 10 ml ethanol is dropwise added over ca. 2 min. The dropping funnel is rinsed with 5 ml ethanol. The resulting solution is allowed to cool. Seeds are added at 50°C and crystallization starts. The suspension is stirred at room temperature over night. The crystallisate is filtered by suction and washed with 30 ml ethanol.
The crystals are dried for 20 h at 80°C and ca. 10 mbar.
Yield: 3.48 g white powder (98 %)
Elemental analysis:
Calc.: 58.59 % C; 5.77 % H; 11.88 % N; 23.75 % O
Found: 58.31 % C; 6.06 % H; 11.73 % N; 23.93 % O
HPLC purity: 99.2% (a)
The XRPD for the adipate is summarized in Table 12. The XRPD shows a strong diffraction peak at 14.8°.

**Table 12. Powder X-Ray Diffraction Peaks - Adipate**

| 2-theta (deg) | d-spacings (Å) | Relative intensity |
|---|---|---|
| 14.2 | 6.20 | M |
| 14.5 | 6.07 | M |
| 14.8 | 5.96 | S |
| 16.2 | 5.46 | L |
| 18.8 | 4.70 | S |
| 19.2 | 4.60 | S |
| 19.9 | 4.45 | M |
| 20.3 | 4.36 | S |
| 24.6 | 3.61 | M |
| 25.7 | 3.46 | S |
| 26.3 | 3.38 | M |
| 26.8 | 3.31 | M |
| 28.5 | 3.12 | L |
| 29.2 | 3.05 | L |

FT-IR Main IR bands: 3100; 1714; 1649; 1568; 1516; 1459; 1409; 1394; 1282; 1215; 1193; 1182; 1173; 1150; 1090; 1053; 808; 767 cm⁻¹

### Example 11 Glutarate Form A

A suspension of 3.0 g camostat base (7.53 mmoles) in 50 ml ethanol is heated at 65°C. Then a solution of 1.0 g glutaric acid (7.53 mmoles) in 10 ml ethanol is added dropwise over ca. 2 min. The dropping funnel is rinsed with 5 ml ethanol. The resulting solution is allowed to cool. Seeds are added at 60°C and crystallization starts. The suspension is stirred at room temperature over night. The crystallisate is filtered by suction and washed with 30 ml ethanol.
The crystals are dried for 20 h at 80°C and ca. 10 mbar.
Yield: 3.43 g white powder (98 %)
Elemental analysis:
Calc.: 58.18 % C; 5.64 % H; 12.06 % N; 24.11 % O
Found: 58.27 % C; 5.86 % H; 11.76 % N; 23.88 % O
HPLC purity: 99.3% (a)
The XRPD for the glutarate is summarized in Table 13. The XRPD shows a strong diffraction peak at 19.1°.

**Table 13. Powder X-Ray Diffraction Peaks - Glutarate**

| 2-theta (deg) | d-spacings (Å) | Relative intensity |
|---|---|---|
| 14.2 | 6.21 | M |
| 14.6 | 6.03 | S |
| 15.1 | 5.84 | M |
| 18.8 | 4.70 | S |
| 19.1 | 4.63 | S |
| 20.7 | 4.26 | M |
| 24.6 | 3.60 | M |
| 25.7 | 3.46 | S |
| 26.3 | 3.38 | L |
| 26.8 | 3.31 | L |
| 27.9 | 3.19 | L |
| 28.5 | 3.11 | L |
| 29.1 | 3.06 | L |

FT-IR Main IR bands: 3100, 1744, 1715, 1649; 1569; 1516; 1459; 1409; 1394; 1282; 1215; 1193; 1182; 1173; 1149; 1089; 1053; 805; 766 cm⁻¹

### Example - Camostat Base Form II

This form was obtained after equilibration in water or in Ethanol/ water (1/1) at 25°C, followed by filtration.

The X-ray diffraction pattern for camostat base Form II is summarized in Table 14. The XRPD shows a strong diffraction peak at 12.5°.

**Table 14. Powder X-Ray Diffraction Peaks Camostat Base Form II**

| 2-theta (deg) | d-spacings (Å) | Relative intensity |
|---|---|---|
| 8.7 | 10.10 | L |
| 12.5 | 7.03 | S |
| 14.4 | 6.13 | L |
| 16.1 | 5.48 | M |
| 16.6 | 5.31 | S |
| 17.4 | 5.06 | L |
| 19.7 | 4.48 | S |
| 21.2 | 4.17 | M |
| 22.4 | 3.96 | M |
| 22.8 | 3.88 | M |
| 23.3 | 3.80 | M |
| 23.6 | 3.76 | M |
| 25.2 | 3.52 | L |
| 26.2 | 3.39 | L |
| 26.6 | 3.34 | S |
| 27.7 | 3.21 | M |
| 29.8 | 2.99 | L |
| 33.9 | 2.63 | L |

FT-IR Main IR bands: 3435; 3364; 1725; 1660; 1616; 1507; 1445; 1309; 1266; 1250; 1202; 1165; 1139; 1064; 894; 813; 797; 710 cm-1

### Pharmaceutical Formulation Examples

The following are non-limiting examples of formulations of the present invention suitable for nebulisation. For the avoidance of doubt, 'camostat' as used below refers to camostat free base in any form, e.g. Form II, or a camostat salt. Suitably the camostat salt is camostat mesylate or a salt selected from camostat hydrogensuccinate, camostat succinate, camostat phosphate, camostat acetate, camostat hydrogentartrate hemihydrate, camostat glycolate, camostat glycolate hemihydrate, camostat hippurate, camostat 1-hydroxy-2-naphthoate (xinafoate), camostat adipate and camostat glutarate.

### Example 1

Camostat 25mg
Lactose 125mg
sodium saccharine 25mg reconstitution with 5ml isotonic saline

### Example 2

Camostat 2.5mg
Lactose 125mg
sodium saccharine 2.5mg reconstitution with 5ml isotonic saline

### Example 3

Camostat 25mg
Lactose 125mg
reconstitution with 5ml isotonic saline + sodium saccharine 25mg

### Example 4

Camostat 2.5mg
Lactose 125mg
reconstitution with 5ml isotonic saline + sodium saccharine 2.5mg

## Claims

1. A pharmaceutically acceptable salt form of camostat selected from camostat hydrogensuccinate, camostat succinate, camostat phosphate, camostat acetate, camostat hydrogentartrate, camostat glycolate, camostat hippurate, camostat 1-hydroxy-2-naphthoate (xinafoate), camostat adipate and camostat glutarate, or a solvate thereof.

2. A salt according to claim 1 selected from camostat hydrogensuccinate, camostat succinate, camostat phosphate, camostat acetate, camostat hydrogentartrate hemihydrate, camostat glycolate, camostat glycolate hemihydrate, camostat hippurate, camostat 1-hydroxy-2-naphthoate (xinafoate), camostat adipate and camostat glutarate.

3. Use of a salt according to claim 1 or 2 in the manufacture of a medicament for the treatment of cystic fibrosis or COPD.

4. A process for forming a lyophilised form of a pharmaceutically acceptable salt of camostat comprising (i) forming an aqueous solution comprising the camostat salt, and (ii) subjecting the aqueous solution to freeze-drying.

5. A process according to claim 4 wherein the aqueous solution includes an excipient selected from lactose, mannitol and glucose.

6. A process according to claim 4 or 5 where the camostat salt is camostat mesylate.

7. A process according to claim 4 or 5 where the camostat salt is selected from camostat hydrogensuccinate, camostat succinate, camostat phosphate, camostat acetate, camostat hydrogentartrate, camostat glycolate, camostat hippurate, camostat 1-hydroxy-2-naphthoate (xinafoate), camostat adipate and camostat glutarate, or a solvate thereof.

8. A process for forming a nebuliser formulation of a pharmaceutically acceptable salt of camostat comprising the process according to any one of claims 4-7 and the additional step (iii) reconstituting the lyophilised form in an aqueous vehicle.

9. A lyophilised form of a pharmaceutically acceptable salt of camostat prepared by a process according to any one of claims 4-7.

10. A nebuliser formulation prepared by a process according to claim 8.

11. A nebuliser formulation according to claim 10 additionally comprising one or more excipients selected from co-solvents, preservatives, chelating agents, pH and tonicity regulators and surfactants.

12. Use of a nebuliser formulation according to claim 10 or 11 in the manufacture of a medicament for the treatment of cystic fibrosis or COPD.

13. A taste-masked formulation comprising camostat or a pharmaceutically acceptable salt thereof and a taste-masking excipient.

14. A taste-masked formulation according to claim 13, including one or more excipients selected from saccharine sodium, aspartame, menthol and polyalcohols such as sorbitol and xylitol.

15. A taste-masked formulation according to claim 13 or 14, wherein camostat is in a salt form selected from camostat mesylate, camostat hydrogensuccinate, camostat succinate, camostat phosphate, camostat acetate, camostat hydrogentartrate, camostat glycolate, camostat hippurate, camostat 1-hydroxy-2-naphthoate (xinafoate), camostat adipate and camostat glutarate, or a solvate thereof.

16. Use of a taste-masked formulation according to any one of claims 13-15 in the manufacture of a medicament for the treatment of cystic fibrosis or COPD.

17. A nebuliser formulation comprising a pharmaceutically acceptable salt form of camostat and one or more excipients selected from co-solvents, preservatives, chelating agents, pH and tonicity regulators and surfactants.

18. A formulation according to claim 17 where the excipients are selected from one or more of propylene glycol, ethanol, glycerin, benzalkonium chloride, sodium edentate, ascorbic acid, citric acid, hydrochloric acid, sodium hydroxide, sodium chloride, oleic acid, and lecithin.

19. A formulation according to claim 17 or 18 further comprising one or more taste-masked excipients selected from saccharine sodium, aspartame, menthol and polyalcohols such as sorbitol and xylitol.

20. A formulation according to any one of claims 17-19 where the salt form of camostat is selected from camostat mesylate, camostat hydrogensuccinate, camostat succinate, camostat phosphate, camostat acetate, camostat hydrogentartrate, camostat glycolate, camostat hippurate, camostat 1-hydroxy-2-naphthoate (xinafoate), camostat adipate and camostat glutarate, or a solvate thereof.

21. Use of a formulation according to any one of claims 17-20 in the manufacture of a medicament for the treatment of cystic fibrosis or COPD.

22. Use according to claim 21 where the salt form of camostat is selected from camostat mesylate, camostat hydrogensuccinate, camostat succinate, camostat phosphate, camostat acetate, camostat hydrogentartrate, camostat glycolate, camostat hippurate, camostat 1-hydroxy-2-naphthoate (xinafoate), camostat adipate and camostat glutarate, or a solvate thereof.

23. Camostat free base Form II, **characterized by** a strong XPRD peak at 12.5°.
